# EUROPÄISCHE PATENTSCHRIFT

(11) **EP 1 035 802 B1**
(45) Veröffentlichungstag und Bekanntmachung des Hinweises auf die Patenterteilung: **07.04.2004**
(21) Anmeldenummer: 98950033.5
(22) Anmeldetag: 15.09.1998
(51) Int. Cl.: A61B 17/15

(54) **SYSTEM ZUR REKONSTRUKTION DER TORQUIERTHEIT ZWISCHEN DEM NATÜRLICHEN KNIE UND DEM BEREICH DER NATÜRLICHEN HÜFTE**
SYSTEM FOR RECONSTRUCTING TORQUE BETWEEN THE NATURAL KNEE AND AN AREA OF THE NATURAL HIP
SYSTEME DE RESTAURATION DU COUPLE ENTRE LE GENOU NATUREL ET LA ZONE HANCHE NATURELLE

(30) Priorität: 01.12.1997 DE 19753236
(43) Veröffentlichungstag der Anmeldung: 20.09.2000
(73) Patentinhaber: ESKA Implants GmbH & Co., 23556 Lübeck (DE)
(72) Erfinder: GRUNDEI, Hans, D-23558 Lübeck (DE)
(74) Vertreter: Fuchs Mehler Weiss & Fritzsche
(86) Internationale Anmeldenummer: PCT/EP1998/005837
(87) Internationale Veröffentlichungsnummer: WO 1999/027860

(56) Entgegenhaltungen:
- EP-A- 0 709 061
- US-A- 5 364 401

## Beschreibung

Die vorliegende Erfindung betrifft ein System zur Rekonstruktion der natürlichen Torquiertheit zwischen dem natürlichen Knie und dem Bereich der natürlichen Hüfte in einer Anordnung zwischen einem künstlichen Kniegelenk und dem Bereich der natürlichen Hüfte nach teilweiser Resektion der natürlichen Kondylen des Kniegelenks und Anlage eines Femurteils des künstlichen Kniegelenks von bestimmter Größe an den Resektionsflächen des Femurknochens.

Unter Torquiertheit wird em Insichyerdrehen des Femurknochens verstanden, das zu einem personenindividuellen Winkel zwischen dem Kniegelenk einerseits und dem Schenkelhals und Hüftkopf andererseits führt, was das Laufverhalten und das Laufbild maßgeblich mit beeinflußt.

Erweist sich nun der Ersatz des natürlichen Kniegelenkes, beispielsweise infolge eines Tumorbefalls, durch ein künstliches Totalersatzimplantat als notwendig, müssen die natürlichen Gelenkteile teilreseziert werden und durch Implantatkomponenten ersetzt werden. Wird die Femurresektion in herkömmlicher Weise durchgerührt, kann es der Fall sein, daß die Femurktimponente des künstlichen Kniegelenks in einem veränderten Winkel zu dem Hüftbereich steht als das natürliche Kniegelenk vor der Resektion, d.h. also, daß die natürliche Torquiertheit sich nach der Operation nicht mehr im Gesamtsystem des Femurs wiederfindet. Ein verändertes Laufverhalten und ein verändertes Laufbild des Patienten ist die unmittelbare Folge.

Bevor ein Femurteil an dem Femur angesetzt werden kann, wird generell zunächst eine horizontale Anlagefläche unter Resektion des Gleitteiles der Kondylen mittels einer entsprechenden Säge hergestellt. Es fehlen hiernach noch der Ventral- und Dorsalschnitt sowie die beiden die letzteren mit der horizontalen Anlagefläche verbindenden Diagonalschnitte. Die US-A-5 346 401 beschreibt ein System zur Herstellung der Aussenrotation eines Schneideblockes für ein künstliches Kniegelenk mit Hilfe eines Keils. Mit Herstellung dieser Aussenrotation sollen günstigere Belastungverhältnisse für die Patella geschaffen werden und die auf die Patellarsehne einwirkenden Kräfte reduziert werden. Als Bezugstinie für den Keil dienen die hinteren Kondylen.

Eine Vorrichtung zur Festlegung von Resektionsflächen am Femur und an der Tibia zur Vorbereitung einer Implantation einer Kniegelenkstotalendoprothese ist bekannt aus der DE-C-44 23 717. In dieser Druckschrift ist der Schwerpunkt darauf gelegt, daß der Ventral- und der Dorsalschnitt am Femur exakt parallel vorgenommen wird zu der frontalen Resektionsfläche an der Tibia. Diese Druckschrift gibt im Hinblick auf einen etwaigen Verdrehwinkel bei der Herstellung der Resektionsflächen keinen Hinweis, so daß es bei unsachgemäßer Handhabung der in dieser Druckschrift offenbarten Vorrichtung dazu kommen kann, daß die Torquiertheit nach der Operation gegenüber vorher eine völlig andere ist. Dies läßt sich jedoch nicht ohne weiteres korrigieren, zumal wenigstens ein weiterer schwerer Eingriff vonnöten wäre.

Bei dem beschriebenen System wird davon ausgegangen, daß der Frontalschnitt an der Tibia horizontal durchgeführt wird. Die Horizontalebene wird dann beispielsweise durch einen Paralleler gemäß dem deutschen Patent 44 23 717 parallel verschoben und am Femurknochen abgebildet, so daß auch der frontale femorale Resektionsschnitt horizontal liegt.

Es ist vorgesehen, daß das System eine Nagellehre mit einem exakt quaderförmigen Anlageblock aufweist, der an seiner von ventral gesehenen Stirnfläche einen davon abragenden, femurwärts weisenden Schenkel aufweist, der an seinem Ende einen Femurkontaktfühler in Form eines senkrecht auf dem Schenkel stehenden Bolzens zur ventral gelegenen punktförmigen Auflage auf dem Femur hält, und an seiner von dorsal gesehenen Stirnfläche mindestens eine davon abragende, femurwärts weisende Anschlagslasche zur jeweils dorsal gelegenen punktförmigen Anlage an beiden dorsalen Kondylenrollen aufweist, wobei der Anlageblock durch Bohrungspaare durchsetzt ist, deren Anordnung die jeweilige Größe des Femurteils repräsentiert, durch welche Fixationsnägel setzbar sind, welche den Anlageblock in seiner Lage auf dem Femur sichert, und wobei der Anlageblock von diesem unter Zurückbelassung der Fixationsnägel abziehbar ist, sowie eine Sägelehre mit identischer Grundform wie der Anlageblock der Nagellehre mit identischer Anordnung der Bohrungspaare in dem Anlageblock, welcher auf die Fixationsnägel aufsetzbar ist, so daß ihre Stirnseiten die übrigen Resektionsebenen festlegen.

Verbindendes Element zwischen der Nagellehre und der Sägelehre sind demnach die Fixationsnägel, die in Bohrungspaare sowohl im Anlageblock als auch in der Sägelehre - je nach Größe des Gelenkes - passen, so daß die Lage der Nagellehre übertragen wird auf die Lage der Sägelehre auf dem Femurstumpf. Voraussetzung für die Anwendung des Systems ist zunächst die Herstellung einer horizontalen Anlagefläche für den Femurteil des künstlichen Kniegelenkes. Hierbei werden die Gleitpartien der natürlichen Kondylen entfernt. In ca. 90 % der Operationsfälle bleiben die dorsalen Kondylenrollen unversehrt und können somit als Bezugspunkt für die Feststellung der Torquiertheit herangezogen werden. Ausgangspunkt der Überlegung bei dem System ist es, daß erst eine stabile Dreipunktlage in der Lage ist, ein stabiles System zur Abbildung der Torquiertheit auszubilden. Zwei der drei Punkte werden festgelegt durch die dorsal gelegenen Berührungspunkte der erwähnten Anschlagslasche an den beiden Kondylenrollen.

Der noch fehlende dritte Punkt für die Dreipunktauflage der Nagellehre wird festgelegt durch den Femurkontaktfühler, der auf der gegenüberliegenden Seite, also ventral gelegen, für einen punktförmigen Berührungspunkt mit dem Femur sorgt.

Der Operateur muß also zunächst - nachdem die Größe des zu implantierenden Femurteils eindeutig festgelegt worden ist - die horizontale Anlagefläche am Femur herstellen und dann die Nagellehre so auf den Femurstumpf setzen, daß eine stabile Dreipunktauflage der Nagellehre auf dem Femurstumpf zustande kommt kraft des Femurkontaktfühlers und der wenigstens einen Anschlagslasche für die beiden Kondylenrollen.

Nach Herstellung der stabilen Dreipunktlage der Nagellehre fixiert der Operateur diese auf der horizontalen Anlagefläche mittels zweier Fixationsnägel, die er durch das der Größe des Implantates entsprechende Bohrungspaar durch den Anlageblock in den Femurknochen schlägt. Danach wird die Nagellehre unter Zurückbelassung der Fixationsnägel vom Femur abgezogen und die erwähnte Sägelehre auf die noch im Femur steckenden Fixationsnägel gesetzt. Die Abbildung der Torquiertheit erfolgt aufgrund der identischen Grundform der Sägelehre wie die Nagellehre und der identischen Anordnung der Bohrungspaare in der Sägelehre sowie in der Nagellehre. Nach Aufsetzen der Sägelehre auf die Fixationsnägel und ggf. einer weiteren Fixierung der Sägelehre werden nun die übrigen Resektionsschnitte entsprechend der Vorgabe der Sägelehre durchgeführt. Im einzelnen handelt es sich hierbei um den dorsalen Schnitt, den ventralen Schnitt sowie um die beiden diagonalen Schnitte, derart, daß die so hergestellten Diagonalschnittflächen die horizontale Anlagefläche verbindet mit der dorsalen bzw. ventralen Resektionsfläche. Sodann kann das Femurteil des künstlichen Kniegelenkes mit den entsprechenden Anlageflächen am Femur fixiert werden, sei es zementlos oder mittels Knochenzement.

Ein typisches Femurteil für ein künstliches Kniegelenk mit entsprechenden Anlageflächen zeigt im übrigen beispielsweise die DE-A-41 41 757.

Als Ausgangspunkt für die vorliegende Erfindung kommt ein weiteres hinzu:

Die laterale Kondyle des Femurs liegt höher als die mediale Kondyle. Die gedachte Verbindungslinie von lateral nach medial zwischen den beiden Kondylen ist demnach gegenüber der Horizontalen geneigt. Dies führte zu der Überlegung, daß auch die Resektionsfläche an der Tibia eine gegenüber der Horizontalen geneigte Fläche sein sollte, um möglichst natürliche Bedingungen im künstlichen Kniegelenk zu erzeugen. Sodann stellt sich freilich die Frage, wie das beschriebene System noch verwendet werden kann, da dieses von der Vornahme von Horizontalschnitten ausgeht.

Zum besseren Verständnis wird Bezug genommen auf die Zeichnungsfiguren 1 bis 5, welche das beschriebene System darstellen. Dabei zeigt:
- Fig. 1: eine Frontalansicht auf die Nagellehre, aufgesetzt auf die Kondylen des natürlichen Kniegelenkes,
- Fig. 2: die Ansicht auf das teilresezierte natürliche Kniegelenk von medial mit aufgesetzter Sägelehre,
- Fig. 3: die Frontalaufsicht auf die am Femurstumpf befestigte Sägelehre,
- Fig. 4: die Ansicht von medial auf die Nagellehre, fixiert auf der horizontalen Anlagefläche des Femurs, und
- Fig. 5: die Ansicht von medial auf das natürliche Kniegelenk, vorbereitet zur Herstellung der horizontalen Anlagefläche.

In den Figuren sind gleiche Bestandteile stets mit denselben Bezugszeichen versehen.

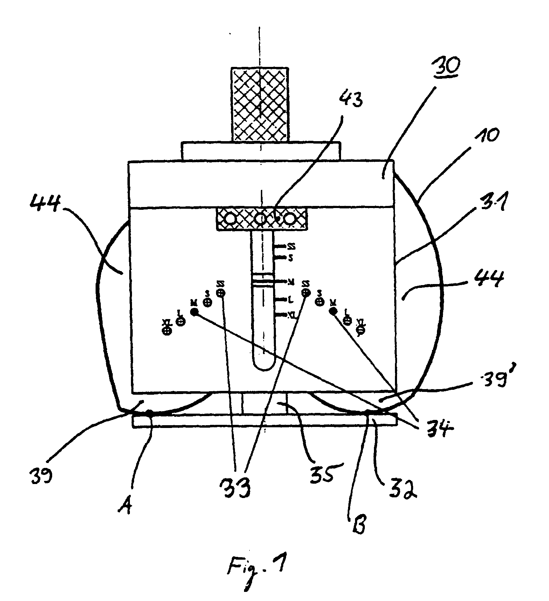

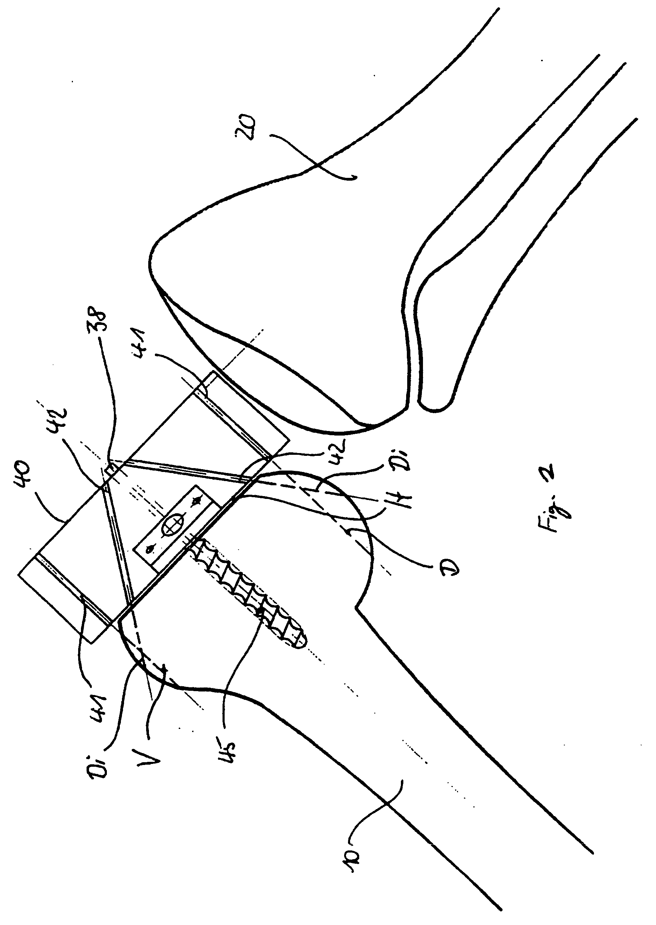

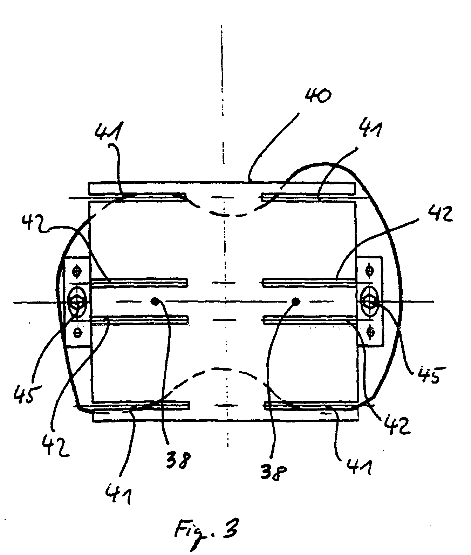

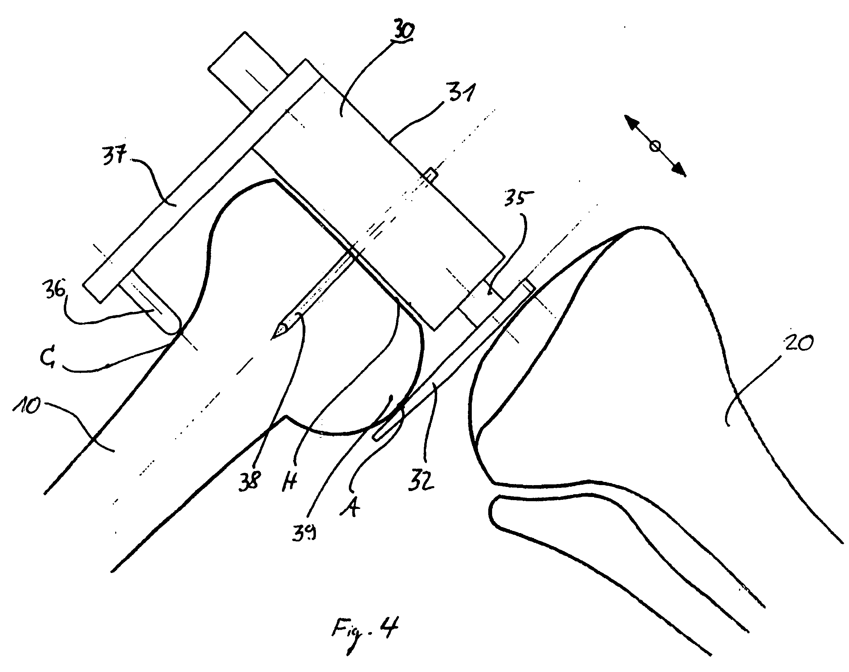

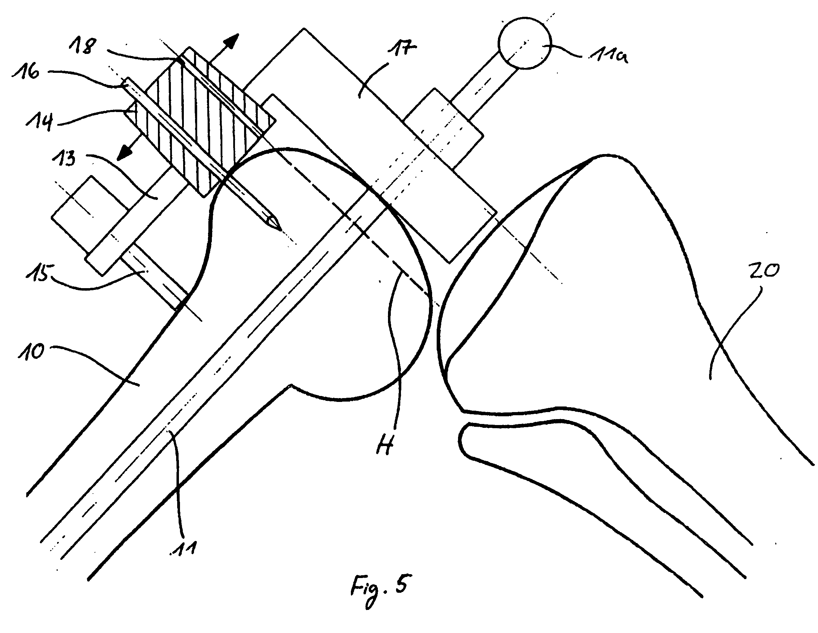

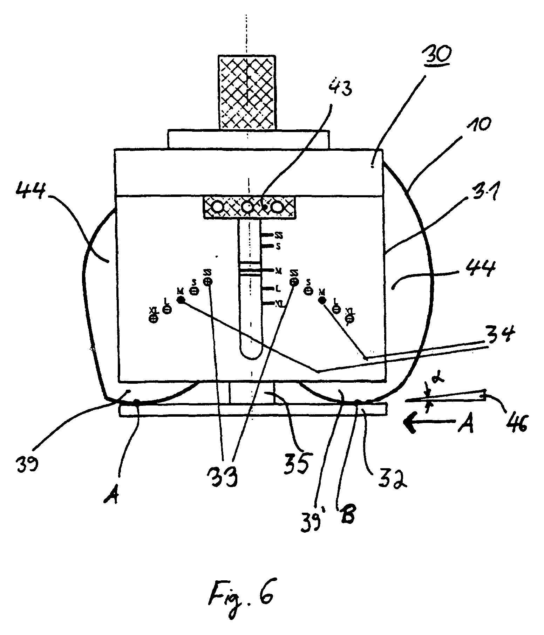

Der konstruktive Aufbau der Nagellehre 30 (Fig. 1) sowie der Sägelehre 40 (Figuren 2 und 3) wird nachfolgend kurz anhand der Beschreibung der Figuren 1 bis 3 erläutert. Die Anwendung des beschriebenen Sytems soll anhand der Beschreibung der Figuren 4 und 5 verdeutlicht werden.

Fig. 1 zeigt die Frontalaufsicht auf die Nagellehre 30, wie sie die Kondylen 44 des natürlichen Kniegelenks am distalen Ende des Femurs 10 übergreift. Die Nagellehre 30 besteht im wesentlichen aus einem exakt quaderförmigen Anlageblock 31. An der dorsal gelegenen Seite (in Fig. 1 also unten) ist eine Anschlagslasche 32 gerührt, welche die hinteren Kondylenrollen 39, 39' lediglich an den Punkten A und B berührt. Vorliegend ist die Anschlagslasche 32 mit dem Anlageblock über eine Gewindespindel 35 verbunden, welche mit einer Rändelschraube 43 als Getriebemechanismus so zusammenarbeitet, daß der Abstand der Anschlagslasche 32 vom Anlageblock 31 variiert werden kann, derart, daß die Nagellehre 30 nach Art einer Schiebelehre zur Größenbestimmung und -festlegung des Femurteils des künstlichen Kniegelenkes dient. Die Punkte A und B bilden zwei Punkte für die anvisierte stabile Dreipunktanlage. Der dritte Punkt C (Fig. 4) ist der Berührungspunkt des Femurkontaktfühlers 36 der Nagellehre 30. Der Femurkontaktfühler 36 ist ausgebildet als ein senkrecht auf einem von der ventral gelegenen Stirnfläche des Anlageblockes 31 abragenden, femurwärts weisenden Schenkel 37 stehender Bolzen. Der Operateur muß nach Festlegung der Größe des Femurteils des Implantates die Nagellehre in eine stabile Dreipunktlage mit den Auflagepunkten A, B und C bringen. Erst dann ist ein Bezug hergestellt zur Torquiertheit zwischen dem Knie und dem Hüftbereich des Patienten.

Der Anlageblock 31 ist durchsetzt mit einer Reihe von Bohrungspaaren 33, 34, von denen jedes Paar einer bestimmten Größe eines Femurteils entspricht.

Nach Auffinden der stabilen Dreipunktlage der Nagellehre 30 wird nach vorangegangener Größenbestimmung und Zuordnung des betreffenden Bohrungspaares 33 und 34 in jede der beiden Bohrungen des betreffenden Bohrungspaares jeweils ein Fixationsnagel 38 gesetzt und in den Femur 10 geschlagen. Die Fixationsnägel 38 sind dabei so ausgebildet, daß die Nagellehre 30 ohne weiteres unter Zurücklassung der Fixationsnägel 38 im Femur 10 von diesem abgezogen werden kann. Die Fixationsnägel 38 weisen also keinen verdickten Kopf auf.

Sodann kommt die Sägelehre 40 (Fig. 2) zum Einsatz. Die Sägelehre 40 verfügt über dasselbe Bohrungspaarlochmuster wie die Nagellehre 30. Die Sägelehre 40 kann demnach nun auf die im Femurknochen verbliebenen Fixationsnägel 38 gesetzt werden. Da die Größenbestimmung bereits vorab vorgenommen worden ist, liegen die Schlitze 41 zur Herstellung der senkrechten Schnitte ventral und dorsal sowie die Schlitze 42 für die Diagonalschnitte automatisch in der richtigen Lage zum Femur, wobei die Torquiertheit aufgrund der Anwendung des Systems erhalten bleibt. Durch Hindurchführen eines oszillierenden Sägeblattes kann der Ventralschnitt V, der Dorsalschnitt D und die beiden Diagonalschnitte Di vorgenommen werden. Vorliegend ist die Sägellehre 40 noch durch zwei zusätzliche Fixationsschrauben 45 (Fig. 3) am Femur arretiert, so daß aufgrund des Sägevorganges keine Verlagerung der Sägelehre 40 stattfinden kann.

Die Anwendung des Systems wird nachfolgend kurz beschrieben:

Zu Beginn der Resektion wird zunächst die horizontale Anlagefläche H (Fig. 5) hergestellt. Hierzu wird ein Anlageblock 17 frontal auf den Femur 10 gesetzt. Ein den Anlageblock 17 durchsetzender Führungsspieß 11 mit Hantel 11a greift in den Markraum des Femurs 10. Auf dem Führungsspieß wird der Anlageblock 17 sozusagen aufgefädelt. Ein femurwärts weisender Schenkel 13 ist mit einem Femurkontaktfühler 15 versehen. Auf dem Schenkel 13 ist eine Sägelehre 14 verfahrbar in Längsrichtung des Schenkels 13. Ein Schlitz 18 in der Sägelehre 14 gibt die Richtung und Lage der horizontalen Anlagefläche H vor. Durch den Schlitz 18 hindurchgeführt wird eine oszillierende Knochensäge. Vor der Anwendung der Säge allerdings wird die Sägelehre 14 mit Fixationsnägeln 16 am Femur 10 gesichert. Sodann wird der Anlageblock 17 und der Führungsspieß ebenso wie der Femurkontaktfühler 15 entfernt und der Schnitt für die horizontale Anlagefläche H durchgeführt.

Nach einer derartigen Vorbereitung kommt das System zum Einsatz, wie anhand der Fig. 4 veranschaulicht wird. Vorliegend ist die Nagellehre 30 - wie ausgeführt - mit einem Getriebemechanismus 35 und 43 (Fig. 1) ausgestattet, so daß sie nach Art einer Schiebelehre zur Größenbestimmung des Femurteils des künstlichen Kniegelenkes herangezogen werden kann. Hierzu wird die Nagellehre 30 zunächst wieder in die stabile Dreipunktlage A, B und C gebracht, so daß also die Anschlagslasche 32 die hinteren Kondylenrollen 39 und 39' jeweils an nur einer Stelle berührt, ebenso wie der Femurkontaktfühler 36 den Femur 10 auf der gegenüberliegenden Seite.

Die stabile Dreipunktlage wird nicht beeinträchtigt durch einen direkten Kontakt des Anlageblockes 31 mit der horizontalen Anlagefläche A. Nach dem Auffinden der stabilen Dreipunktlage werden Fixationsnägel 38 in das betreffende Bohrungspaar 33, 34 gesetzt und in den Femur 10 geschlagen, wonach dann die Nagellehre unter Zurückbelassung der Fixationsnägel 38 im Femur 10 abgezogen werden kann und die Sägelehre 40, wie anhand von Fig. 2 bereits beschrieben, auf die Fixationsnägel 38 gesetzt werden kann, wonach die übrigen Resektionsschnitte vorgenommen werden können.

Demgemäß ist es also die Aufgabe der vorliegenden Erfindung, das beschriebene System so weiterzubilden, daß es eine exakte Nachbildung der natürlichen Torquiertheit zwischen dem Knie und der Hüfte abbildet unter der Voraussetzung, daß der Resektionsschnitt an der Tibia kein Horizontalschnitt, sondern vielmehr ein geneigter Schnitt ist, der in einem Winkel zwischen 3 bis 5° gegenüber der Horizontalen von lateral nach medial geneigt ist.

Gelöst wird diese Aufgabe durch das System gemäß dem Anspruch 1. Vorteilhafte Weiterbildungen sind in den Unteransprüchen angegeben.

Dementsprechend wird vorgeschlagen, daß das beschriebene System zusätzlich einen Keil mit einem Keilwinkel α aufweist, der zwischen die laterale Kondylenrolle und die Anschlagslasche der Nagellehre schiebbar ist zur Kompensation eines Keilwinkels, in dem der horizontale Tibiaschnitt von der Kniespaltachse abweicht.

Operativ geht man nun so vor, daß zunächst ein Resektionsschnitt an der Tibia vorgenommen wird, welcher im Bereich der schon erwähnten 3 bis 5° von der Horizontalen abweicht, und zwar von lateral nach medial abfallend. Exakt parallel zu der Tibiaresektionsfläche wird die Femurresektionsfläche frontal gesetzt, und zwar unter vorheriger Zuhilfenahme beispielsweise eines Parallelers gemäß dem deutsche Patent 44 23 717. Sodann kommt die Nagellehre gemäß der Hauptanmeldung zum Einsatz. Allerdings wird zur Kompensation des Neigungswinkels nun am lateralen Berührungspunkt zwischen der lateralen Kondylenrolle und der Anschlagsfläche der erfindungsgemäße Keil geschoben, so daß sich ein exaktes Abbild der natürlichen Verhältnisse ergibt.

Während also gemäß dem deutschen Patent 44 23 717 von zusammenhängenden horizontalen Frontalresektionen sowohl der Tibia als auch des Femurs ausgegangen wird, wird bei der Anwendung des vorliegenden, um den Keil ergänzten Systems, von getrennten Resektionsvorgängen an der Tibia und an der Femurseite ausgegangen, welche den natürlichen Verhältnissen noch näher kommen.

Femurseitig wird dorsal-medial daher mehr Knochenmaterial abgesägt als dorsal-lateral. Insgesamt stellt sich das künstliche Kniegelenk, das an bzw. in die mit dem erfindungsgemäßen System präparierten Tibia- und Femurknochen implantiert worden ist, als noch spannungsfreier dar, als dies bislang möglich war. Der Bewegungsablauf des künstlichen Kniegelenks wird hierdurch - selbstverständlich abhängig vom verwendeten Typ des künstlichen Kniegelenks - optimiert.

Vorteilhaft liegt der Keilwinkel α im Bereich von 3 bis 5°. Dieser Winkel korreliert mit dem Resektionswinkel an der Tibia. 3° wird man bei kleinen Knien wählen, 5° hingegen bei größeren Knien. Sämtliche Werte zwischen den beiden Eckwerten sind möglich.

Gemäß einer vorteilhaften Weiterbildung ist vorgesehen, daß die wenigstens eine dorsal gelegene Anschlagslasche und der ventral gelegene Schenkel mittels eines Getriebemechanismus verbunden sind, mittels dessen ihr Abstand voneinander variiert werden kann.

In dieser Ausführungsform kann die Nagellehre vorab auch zur Größenbestimmung des Femurteils verwendet werden. Im Prinzip funktioniert die Nagellehre dann wie eine Schiebelehre. Der Operateur betätigt den Getriebemechanismus zunächst so, daß die Anschlagslasche und der Schenkel so weit voneinander entfernt sind, daß die Nagellehre ohne weiteres über die Kondylen des natürlichen Kniegelenks gesetzt werden kann, woraufhin der Operateur dann den Getriebemechanismus so betätigt, daß der genannte Abstand verringert wird, so lange, bis die Anschlagslasche und der Femurkontaktfühler den Femurknochen berühren. Eine Skala am Getriebemechanismus kann dem Operateur sofort Auskunft über die zu implantierende Größe des Femurteils und damit des gesamten Kniegelenks geben. Auch richtet sich später die Auswahl der Sägelehre nach der Größenermittlung des Femurteils, wie bereits oben erwähnt.

Gemäß einer noch weiteren Ausführungsform kann vorgesehen sein, daß dorsal gesehen zwei Anschlagslaschen in Form zweier Schenkel - einer für jede Kondylenrolle - vorgesehen sind, die in einer Ebene liegen. Hierdurch wird eine optische Kontrolle von dorsal gesehen bei Herstellung der stabilen Dreipunktlage möglich, indem der Operateur nämlich ausschließen kann, daß der Anlageblock der Nagellehre auf der resezierten horizontalen Anlagefläche des Femurs zu liegen kommt, so daß bereits hierdurch eine stabile Lage der Nagellehre auf dem Femurknochen die Folge wäre, auch wenn die Schritte für die Abbildung für die Torquiertheit noch nicht eingeleitet worden sind.

Die Erfindung wird anhand eines Ausführungsbeispiels gemäß der Zeichnungsfigur 6 näher erläutert.

Diese zeigt die Frontalaufsicht auf die erfindungsgemäße Nagellehre 30, wie sie die Femurkondylen 44 des natürlichen Kniegelenks am distalen Ende des Femurs 10 übergreift. Die Nagellehre 30 besteht im wesentlichen aus einem exakt quaderförmigen Anlageblock 31. An der dorsal gelegenen Seite (in der Zeichnungsfigur also unten) ist eine Anschlagslasche 32 geführt, welche die hinteren Kondylenrollen 39, 39' lediglich an den Punkten A und B berührt. Vorliegend ist die Anschlagslasche 32 mit dem Anlageblock über eine Gewindespindel 35 verbunden, welche mit einer Rändelschraube 43 als Getriebemechanismus so zusammenarbeitet, daß der Abstand der Anschlagslasche 32 vom Anlageblock 31 variiert werden kann, derart, daß die Nagellehre 30 nach Art einer Schiebelehre zur Größenbestimmung und - festlegung des Femurteils des künstlichen Kniegelenkes dient. Die Punkte A und B bilden zwei Punkte für die anvisierte stabile Dreipunktanlage.

Das vorliegende System weist einen Keil 46 auf, der in die durch den Pfeil A angedeutete Richtung zwischen die Anschlagslasche 32 und die laterale Femurkondyle 39' geschoben wird. Hierdurch wird die laterale Femurkondyle 39' angehoben, da insgesamt eine leichte Rotation um den Punkt A, nämlich dem Berührungspunkt zwischen der medialen Kondyle 39 und der Anschlagslasche 32, ausgeführt wird. Diese Rotationsbewegung bietet die Kompensation für die Neigung der frontalen Resektionsfläche an der Tibia. Der Anlagepunkt B wird also von der Anschlagslasche 32 verlagert hin auf den Keil 46.

Der Winkel α liegt bevorzugt im Bereich von 3 bis 5° und entspricht damit der Neigung der horizontalen Resektionsfläche an der Tibia.

## Patentansprüche

1. System zur Rekonstruktion der natürlichen Torquiertheit zwischen dem natürlichen Knie und dem Bereich der natürlichen Hüfte in einer Anordnung zwischen einem künstlichen Kniegelenk und dem Bereich der natürlichen Hüfte nach teilweiser Resektion der natürlichen Kondylen des Kniegelenks und Anlage eines Femurteils des künstlichen Kniegelenks vorbestimmter Größe an den Resektionsflächen des Femurknochens, aufweisend
- eine Nagellehre (30) mit einem exakt quaderförmigen Anlageblock (31), der an seiner von ventral gesehenen Stirnfläche einen davon abragenden, femurwärts weisenden Schenkel, der an seinem Ende einen Femurkontaktfühler in Form eines senkrecht auf dem Schenkel stehenden Bolzens zur ventral gelegenen punktförmigen Auflage (C) auf dem Femur (10) hält, und an seiner von dorsal gesehenen Stimfläche mindestens eine davon abragende, femurwärts weisende Anschlagslasche (32) zur jeweils dorsal gelegenen punktförmigen Anlage (A, B) an beiden dorsalen Femurkondylenrollen (39, 39') aufweist, wobei der Anlageblock (31) durch Bohrungspaare (33, 34) durchsetzt ist, deren Anordnung die jeweilige Größe des Femurteils repräsentiert, durch welche Fixationsnägel setzbar sind, welche den Anlageblock (31) in seiner Lage auf dem Femur (10) sichert, und wobei der Anlageblock (31) von diesem unter Zurückbelassung der Fixationsnägel abziehbar ist, und
- eine Sägelehre mit identischer Grundform wie der Anlageblock (31) der Nagellehre (30) und mit identischer Anordnung der Bohrungspaare in dem Anlageblock (31), welche auf die Fixationsnägel setzbar ist, so daß ihre Stirnseiten die übrigen Resektionsebenen (ventral, dorsal, diagonal) festlegen, zusätzlich aufweisend einen Keil mit einem Keilwinkel α, der zwischen die laterale Kondylenrolle (39') und Anschlagslasche (32) schiebbar ist zur Kompensation eine Torquiertheit des Femurknochens.

2. System nach Anspruch 1, bei dem der Keilwinkel α im Bereich 3° ≤ α ≤ 5° liegt.

3. System nach Anspruch 1 oder 2, bei dem die dorsal gelegene Anschlagslasche (32) und der ventral gelegene Schenkel (37) der Nagellehre (30) mittels eines Getriebemechanismus (35, 43) verbunden sind, mittels dessen ihr Abstand voneinander variiert werden kann.

4. System nach einem der Ansprüche 1 bis 3, bei dem dorsal zwei Anschlagslaschen in Form zweier Schenkel vorgesehen sind, die in einer Ebene liegen.

## Claims

1. System for reconstruction of the natural twist between the natural knee and the region of the natural hip in an arrangement between an artificial knee joint and the region of the natural hip after partial resection of the natural condyles of the knee joint and bearing of a femur part of the artificial knee joint of predetermined size on the resection surfaces of the femur bone, having
- a nail jig (30) with an exactly cuboid-shaped bearing block (31) which, at its end face seen ventrally, has a limb projecting therefrom and pointing towards the femur, which at its end holds a femur contact sensor in the form of a bolt standing vertically on the limb for the ventrally placed point-like support (C) on the femur (10), and at its end face seen dorsally, has at least one stop plate (32) projecting therefrom and pointing towards the femur for the particular dorsally placed point-like support (A, B) at both dorsal femoral condyle rounds (39, 39'), wherein the bearing block (31) is penetrated by bore hole pairs (33, 34), the arrangement of which represents the particular size of the femur part, through which fixing nails can be placed, which secure the bearing block (31) in its position on the femur (10), and wherein the bearing block (31) can be removed from the latter while leaving behind the fixing nails, and
- a saw jig with identical basic shape as the bearing block (31) of the nail jig (30) and with identical arrangement of the bore hole pairs in the bearing block (31), which can be placed on the fixing nails, so that its end faces fix the remaining resection planes (ventral, dorsal, diagonal), additionally having a wedge with a wedge angle α, which can be pushed between the lateral condyle round (39') and stop plate (32) for compensation of a twist of the femur bone.

2. System according to claim 1, in which the wedge angle α lies in the range 3° ≤ α≤5°.

3. System according to claim 1 or 2, in which the dorsally placed stop plate (32) and the ventrally placed limb (37) of the nail jig (30) are connected by means of a gear mechanism (35, 43), by means of which their distance from one anther may be varied.

4. System according to one of claims 1 to 3, in which two stop plates in the form of two limbs, which lie in one plane, are provided dorsally.

## Revendications

1. Système de reconstruction d'un engrenage naturel entre un genou naturel et la région d'une hanche naturelle dans une disposition entre une articulation de genou artificielle et la région d'une hanche naturelle après résection partielle des condyles naturels de l'articulation du genou et la mise en place d'une partie fémorale de l'articulation artificielle du genou de taille prédéterminée sur la surface de résection de l'os fémoral, présentant
- un implant de broche (30) avec un bloc d'insertion de forme précisément parallélépipédique (31), qui porte dans sa partie frontale en vue antérieure un bras s'étendant en direction du fémur, qui possède à son extrémité une tige de contact fémoral sous la forme d'une cheville montée verticalement sur le bras touchant le point d'appui (C) en position antérieure sur le fémur (10), et qui présente sur sa partie frontale en vue postérieure au moins une butée (32) qui s'étend en direction du fémur et touche le point d'appui (A, B) en position postérieure sur les deux condyles fémoraux postérieurs (39, 39'), dans lequel le bloc d'insertion (31) est traversé par deux rangées de trous (33, 34) dont la disposition dépend de la grandeur de chaque élément fémoral, dans lesquels peuvent s'insérer des vis de fixation, qui assurent la position du bloc d'insertion (31) sur le fémur (10), et dans lequel le bloc d'insertion (31) est amovible de celui-ci grâce au retrait des vis de fixation, et
- un gabarit de sciage de forme fondamentalement identique au bloc d'insertion (31) de l'implant de broche (30) et avec une disposition identique des rangées de trous sur le bloc d'insertion (31), lesquelles peuvent correspondre aux vis de fixation, de sorte que leurs parties frontales bloquent les zones de résection résection (antérieure, postérieure, diagonale), présentant en outre une cale avec un angle à, qui peut s'ajuster entre le condyle latéral (39') et la butée (32) en compensation de l'engrenage de l'os du fémur.

2. Système selon la revendication 1, dans lequel l'angle est situé dans la gamme 3° ≤ α ≤ 5°.

3. Système selon la revendication 1 ou 2, dans lequel la butée en position postérieure (32) est reliée au bras en position antérieure (37) de l'implant de broche (30) au moyen d'un mécanisme d'entraînement (35, 43), grâce auquel la distance entre eux peut être variée.

4. Système selon l'une des revendications 1 à 3, dans lequel sont prévues deux butées postérieures sous la forme d'un double bras, qui existe dans un plan.
